(19) Europäisches Patentamt / European Patent Office / Office européen des brevets

(11) **EP 2 446 906 A1**

(12) **EUROPEAN PATENT APPLICATION**
published in accordance with Art. 153(4) EPC

(43) Date of publication:
**02.05.2012 Bulletin 2012/18**

(21) Application number: **10792106.6**

(22) Date of filing: **22.06.2010**

(51) Int Cl.:
**A61L 27/00** (2006.01)

(86) International application number:
**PCT/JP2010/060571**

(87) International publication number:
**WO 2010/150788 (29.12.2010 Gazette 2010/52)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **25.06.2009 JP 2009151091**

(71) Applicant: **Akita University**
**Akita-shi**
**Akita 010-8502 (JP)**

(72) Inventors:
- **YAMAMOTO, Osamu**
  **Akita-shi**
  **Akita 010-8502 (JP)**
- **FUKUDA, Masayuki**
  **Akita-shi**
  **Akita 010-8543 (JP)**

(74) Representative: **Selden, Deborah Anne**
**Beck Greener**
**Fulwood House**
**12 Fulwood Place**
**London WC1V 6HR (GB)**

(54) **PROCESS FOR PRODUCING TITANIUM-BASED BIOMEDICAL IMPLANT HAVING ZINC-CONTAINING FUNCTIONAL GROUP IMPARTED THERETO, AND TITANIUM-BASED BIOMEDICAL IMPLANT**

(57) The present invention provides a titanium-based material for a bio-implant having a fourth generation function given thereto, by a method for producing a titanium-based material for a bio-implant having a zinc functional group, wherein the method comprises a soaking step in which a base material made of titanium and an alloy thereof is soaked in an alkali solution containing a zinc hydroxide complex.

Fig. 1

**Description**

Technical Field

The present invention relates to a titanium-based material for a bio-implant having a zinc functional group given thereto. In specific, it relates to a method for producing a titanium-based material for a bio-implant having a zinc functional group given thereto that can be used as an implant material in orthopedic surgery and dentistry; and a titanium-based material for a bio-implant.

Background Art

In recent years, with the advent of the aging society and with the change in eating habits, an implant treatment has become widely used, replacing the treatments using an artificial tooth and a dental bridge. In the implant treatment, an artificial tooth root is implanted into the jaw bone; and bioceramics have been actively developed which can be used permanently in the oral cavity as a material for a bio-implant (an implant material) to serve as the artificial tooth root.
A material for a bio-implant used in orthopedic surgery and dentistry is required to bond strongly to the bone without being toxic to the living body. Thus, the following materials have been developed: first generation materials such as alumina and carbon which enable the implant material to function in the living body for a long period of time with least toxicity and with the material property matching the surrounding tissue; second generation materials such as apatite and β-calcium phosphate which enable a material implanted in the living body to bond to the bone directly without using the surrounding fibrous connective tissue; and third generation materials for nucleus formation serving as a scaffold for bone formation by ion exchange. And in recent years, fourth generation materials have been developed, which is permitted to have a function to stimulate the growth, differentiation, and organization of the bone cells from the material implanted inside the living body, at the ionic/molecular level.
The third generation materials are mainly β-calcium phosphate; and bioglasses designed to elute a tiny amount of calcium ion, zinc ion, silicon ion or the like which are necessary for bone formation. Further, studies have been conducted on subjecting titanium metal and an alloy thereof for a conventionally used implant to an alkali heat treatment, to thereby chemically modify the surface thereof with a hydroxyl group, to coat the surface thereof with hydroxyapatite, and to form a layer for ion exchange with calcium ion in the living body (a sodium titanate layer). And from these studies, fine bone formation and satisfactory material-bone adhesion have been confirmed (Patent Documents 1 to 4, and Non-Patent Documents 1 to 4).

Citation List

Patent Literature

Patent Document 1: Japanese Patent Application Laid-Open (JP-A) No. 10-179717
Patent Document 2: JP-A No. 10-179718
Patent Document 3: JP-A No. 2000-116673
Patent Document 4: JP-A No. 2002-102330

Non-Patent Literature

Non-Patent Document 1: Nishiguchi S. et al., Biomaterials, viol.22, pp.2525-2533, 2001
Non-Patent Document 2: Ozeki K. et al., Bio-medical Materials and Engineering, vol.11, pp.63-68, 2001
Non-Patent Document 3: Maxian SH. et al., Journal of Biomedical Materials Research, vol.27, pp.717-728, 1993
Non-Patent Document 4: Hayashi K. et al., Biomaterials, viol.15, pp.1187-1191, 1994

Summary of the Invention

Problems to be Solved by the Invention

The fourth generation materials have been studied with main focus on bioglasses. Titanium is light in weight for metal, and causes an extremely minor allergic reaction, if any. And being metal, titanium has very high strength and toughness compared to bioglasses. However, it has been difficult to allow a titanium-based implant material (titanium and a titanium alloy (Ti-6Al-4V) etc.) to have a fourth generation function.

Accordingly, an object of the present invention is to provide a titanium-based material for a bio-implant in which a titanium base material is permitted to have the fourth generation function.

Means for Solving the Problems

According to the studies conducted by the inventors, the following points are important in developing a fourth generation material for a bio-implant suitable for practical use:

(1) in consideration of the physical strength and low toxicity to the living body, a base material is preferably titanium metal and an alloy thereof:
(2) it is necessary to introduce a functional group for nucleus formation:
(3) it is necessary to be able to elute a tiny amount of metallic ion which stimulates the growth and differentiation of the bone cells.

Taking these points into account, the inventors have developed the fourth generation material for a bio-implant suitable for practical use; and completed the following invention. Amaterial for a bio-implant obtained from the following invention has a fourth generation function (a function to stimulate the growth, differentiation, and organization of the bone cells from the material at the ionic/molecular level).

A first aspect of the present invention is a method for producing a titanium-based material for a bio-implant having a zinc functional group, which comprises a soaking step of soaking a base material made of titanium or an alloy thereof into an alkali solution containing a zinc hydroxide complex.

In the first aspect of the present invention, an $OH^-$ concentration of the alkali solution is preferably 5.0 M or more and 8.0 M or less.

In the first aspect of the present invention, the soaking step is preferably carried out by using the alkali solution having a temperature of 40°C or more and 80°C or less.

In the first aspect of the present invention, the soaking time of the base material into the alkali solution in the soaking step is preferably 60 minutes or more and 72 hours or less.

In the first aspect of the present invention, the zinc functional group is preferably a functional group comprising a divalent zinc atom and a hydroxyl group.

In the first aspect of the present invention, the zinc hydroxide complex is preferably $[Zn(OH)_4]^{2-}$.

A second aspect of the present invention is a titanium-based material for a bio-implant comprising a base material made of titanium or an alloy thereof, wherein a surface of the base material is provided with a zinc functional group.

In the second aspect of the present invention, the surface of the base material made of titanium or an alloy thereof preferably comprises a titanium oxide layer, on which the zinc functional group is provided.

In the second aspect of the present invention, the zinc functional group is preferably a functional group comprising a divalent zinc atom and a hydroxyl group.

Effects of the Invention

According to the present invention, it is possible to produce a fourth generation titanium-based material for a bio-implant which exhibits a strong adhesion to the bone. Further, since titanium or an alloy thereof is used as a base material, the titanium-based material for a bio-implant has high strength and low toxicity to the living body. Furthermore, the titanium-based material for a bio-implant of the present invention can be suited for use as an implant material for orthopedic surgery or dentistry.

Brief Description of the Drawings

Fig. 1 is a schematic diagram showing the surface structure of the titanium-based material for a bio-implant of the present invention.
Fig. 2A is an image of the surface state of a sample of Example 1 by FESEM; and Fig. 2B shows results of analysis by EDX of the surface of the sample of Example 1.
Fig. 3 is a graph showing the surface roughness of the samples of Examples 1 and 2, and Comparative Examples 1 and 2.
Fig. 4 shows TF-XRD patterns for the samples of Example 1 and Comparative Example 1.

Fig. 5 shows XPS spectra of the sample of Example 1.

Fig. 6A shows higher resolution narrow-scan spectra for the Ti of the sample of Example 1; Fig.6B shows the same spectra for the O.

Fig. 7 shows higher resolution narrow-scan spectra for the Zn of the sample of Example 1.

Fig. 8 shows ESCA depth profile of the sample of Example 1.

Fig. 9 is a picture showing the state in which the implants of Examples are inserted into the femur of the rabbit.

Fig. 10 is a graph showing the implant-bone shear strength of Examples 3 and 4, and Comparative Examples 3 and 4.

Fig. 11A is SEM image of the implant surface of Example 3 after the biomechanical testing; and Fig, 11B is the same image for Example 4.

Fig. 12 is a graph showing a concentration of elution of zinc ion, of the sample of Example 1.

Description of the Numerals

10    base material
20    titanium oxide layer

Mode for Carrying Out the Invention

(A base material made of titanium or an alloy thereof)

The base material of the present invention may be a base material made of pure titanium and may also be a base material made of a titanium alloy. Examples of the titanium alloy include an alloy of titanium and Ca, V, Na, Mg, P, Nb, Al, Pt, Ta etc. Specifically, they may be Ti-6Al-4V, Ti-6Al-4VELI, Ti-22V-4Al (for example, "DAT 51" made by Daido Steel Co., Ltd.). Titanium and an alloy thereof have strength and toughness required as metal; is light-weighted for metal; and has very few chances to cause allergic reactions to the living body. Therefore, titanium and an alloy thereof are preferable to form the material for a bio-implant of the present invention.

Further, the surface of the base material may be roughened. It has been reported heretofore that an implant of which the surface is roughened shows stronger bone-bonding compared to an implant of which the surface is smoothed by surface grinding. Examples of the method for roughening the surface of the base material include a mechanical grinding treatment using a sandblast, silica sand wheel, and diamond abrasion.

(An alkali solution containing a zinc hydroxide complex)

The titanium-based material for a bio-implant of the present invention can be formed in one step, in which the above described base material made of titanium or an alloy thereof is soaked in a heated alkali solution containing a zinc hydroxide complex, thereby treating a surface of the base material. In the conventional third generation titanium-based material for a bio-implant, it has been necessary to subject the titanium base material to an alkali heating treatment and a subsequent heat treatment at a high temperature. The titanium-based material for a bio-implant of the present invention may be formed in a simpler and more efficient way that the heat treatment subsequent to the alkali treatment is not required.

An example of the zinc hydroxide complex used in the present invention may be a complex represented by $[Zn(OH)_4]^{2-}$; however, other complexes such as an aqua zinc hydroxide complex $(Zn(H_2O)_n(OH)_m)$ may be contained as a small amount of by-product.

This alkali solution containing the zinc hydroxide complex may be produced, for example, by dissolving $Zn(NO_3)_2 6H_2O$ and NaOH in the distilled water. Sometimes, at this point, $Zn(OH)_2$ precipitates and the solution becomes milky and turbid. However, in this case, by further adding NaOH, $Zn(OH)_2$ is dissolved; thereby a homogeneous transparent alkali solution containing a zinc hydroxide complex can be obtained.

In the present invention, the base material made of titanium or an alloy thereof is soaked in the above described alkali solution containing the zinc hydroxide complex, thereby treating the surface of the base material. The lower limit of the $OH^-$ concentration of the alkali solution in which the base material is soaked is preferably 5.0 M or more; and more preferably 5.5 M or more. The upper limit is preferably 8.0 M or less; more preferably 7.0 M or less; and still more preferably 6.5 M or less. If the $OH^-$ concentration is too low, $Zn(OH)_2$ is likely to precipitate. Further, a $Zn^{2+}$ concentration is preferably 0.2 M or more and 1 M or less; and more preferably 0.3 M or more and 0.7 M or less. Furthermore, a temperature of the alkali solution is preferably 40°C or more and 80°C or less; and more preferably 50°C or more and 70°C or less. The lower limit of the treating time by the alkali solution is preferably 60 minutes or more; more preferably 5 hours or more; and still more preferably 12 hours or more. The upper limit is preferably 72 hours or less; and more preferably 36 hours or less. As for the conventional third generation alkali treatment, approximately 3 days of treating time have been required.

After the above treatment by the alkali solution, post-treatments such as washing and drying are preferably carried out. For example, after the base material is washed with distilled water, it is dried inside an electric furnace; thereby obtaining the titanium-based material for a bio-implant having a zinc functional group, of the present invention.

(A structure of the titanium-based material for a bio-implant having a zinc functional group)

The titanium-based material for a bio-implant of the present invention produced by the above described method, has a zinc functional group on the surface of its base material. The zinc functional group is a functional group comprising a divalent zinc atom and a hydroxyl group; specifically, it may be "-Zn-OH".

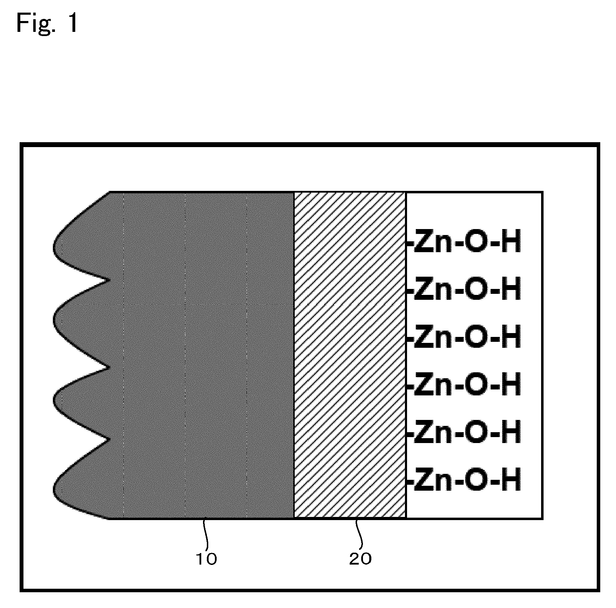

The titanium-based material for a bio-implant of the present invention is not particularly limited as long as it has a zinc functional group on the surface of its base material and is produced by the above described method. When the surface structure of the titanium-based material for a bio-implant of the present invention is shown schematically, it will be the surface structure shown in Fig. 1. In Fig. 1, a base material 10 comprises a titanium oxide layer 20 thereon; and the surface of the titanium oxide layer is provided with a zinc functional group.

The fourth generation material is a material permitted to have a function to stimulate the growth, differentiation, and organization of the bone cells at the ionic/molecular level, by intentionally arranging on the surface of the material, a substance which gives biochemical signals to promote bone formation. Zinc is known as a substance to give such biochemical signals; and the titanium-based material for a bio-implant of the present invention is provided with the fourth generation function, by arranging the zinc on the surface of its base material. In order to effectively promote bone formation, the zinc needs to be released slowly from the surface of the base material. The reason is because highly-concentrated zinc is likely to cause adverse effects such as inhibiting bone formation.

Examples

<Round disc sample evaluation>

(Example 1)

As the base material, a titanium round disc (having a diameter of 10 mm, a thickness of 0.5 mm, and cp-Ti>99.9%; made by Nilaco Co.) was used. The round disc was ultrasonically cleaned with ethanol and water and dried at 70°C for 10 minutes. The alkali solution containing the $[Zn(OH)_4]^{2-}$ complex was prepared by stirring to dissolve 14.85 g of $Zn(NO_3)_2 6H_2O$ (99%; produced by Nacalai Tesque) and 24.00 g of NaOH (96%; produced by Nacalai Tesque) to obtain a 100 ml solution ($Zn^{2+}$=0.5M, $OH^-$=6.0M). At the beginning of the process, $Zn(OH)_2$ precipitated and the solution became milky and turbid. However, by further adding 4.0 g of NaOH, the $Zn(OH)_2$ was dissolved (it is assumed that the reaction of "$Zn(OH)_2 + 2OH^- \rightarrow [Zn(OH)_4]^{2-}$" has occurred) ; thereby a homogeneous transparent alkali solution containing $[Zn(OH)_4]^{2-}$ ion was obtained.

300 ml of the above alkali solution was poured in Teflon (Registered Trademark) beaker. The round disc was put in the beaker and was soaked at 60°C for 24 hours under stirring. This soaking step was carried out to allow the surface of the titanium round disc to have an apatite forming ability and a zinc ion releasing ability.

After the soaking, the round disc was washed with distilled water for one minute and dried in the electric furnace (at 70°C for 30 minutes), thereby obtaining the sample of Example 1.

(Example 2)

The base material to be used was obtained by subjecting a mechanical grinding treatment to the above titanium round disc to increase the surface roughness. A sample of Example 2 was obtained by carrying out the same treatment as that in Example 1, except that the titanium round disc subjected to the surface grinding was used. The mechanical grinding treatment was carried out by using an electric micro grinding machine (made by Urawa Manufacturing Co., Ltd.). The mechanical grinding was carried out by rotating the titanium round disc at 16 rpm and concurrently abrading it with a resin-bonded silica sand wheel (8000 rpm), at room temperature without using a coolant. After the grinding treatment, the titanium round disc was washed with acetone and distilled water in an ultrasonic cleaner.

(Comparative Examples 1, 2)

The titanium round disc of Example 1 was used as a sample of Comparative Example 1, without being subjected to the alkali treatment after washing. Further, the titanium round disc of Example 2, which was subjected to the mechanical grinding treatment was used as Comparative Example 2, without being subjected to the alkali treatment after washing.

The above samples were classified as below. Five pieces were prepared for each of the samples; and the evaluation was conducted by obtaining an average value of these five pieces.

(Example 1) a smoothed surface; with an alkali treatment
(Example 2) a roughened surface; with an alkali treatment
(Comparative Example 1) a smoothed surface; without an alkali treatment
(Comparative Example 2) a roughened surface; without an alkali treatment

(Sample surface evaluation)

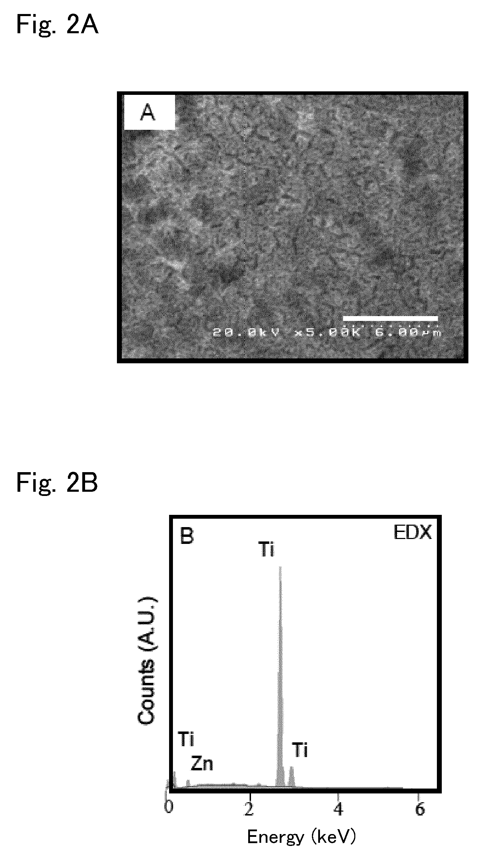

Fig. 2A shows the surface state of the sample of Example 1. The surface state was observed by FESEM (20 kV; made by Hitachi High-Technologies Corporation; S-4500). It can be seen from Fig. 2A that the surface of the sample of Example 1 has a reticulate micro-porous structure.

Fig. 2B shows results of analysis by EDX of the surface of the sample of Example 1. The EDX analysis was conducted by FESEM equipped with the EDX (made by HORIBA; EMAX-7000). It was found from the results that zinc of approximately 2 atom % existed on the surface of the sample.

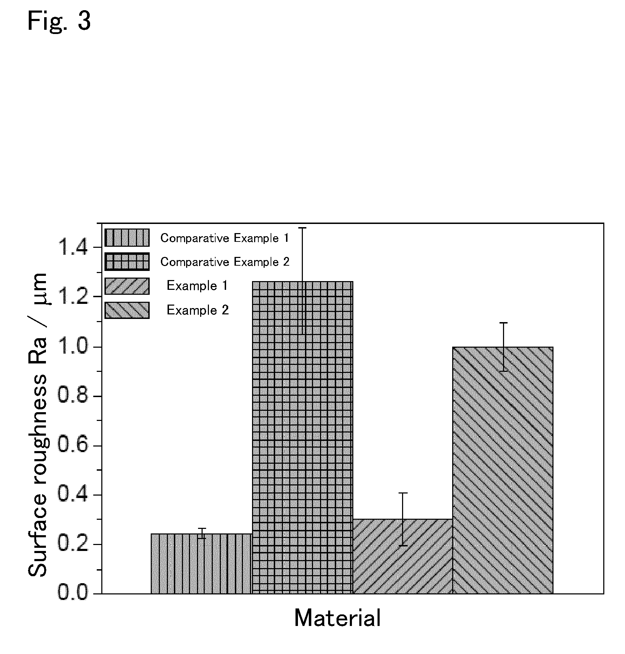

Fig. 3 shows the surface roughness of the samples obtained in Examples 1 and 2, and Comparative Examples 1 and 2. The surface roughness was measured by using a contour-measuring instrument (made by Tokyo Seimitsu Co., Ltd.; SURFCOM 300A).

The surface characteristics measured were digitalized; and the centerline average (Ra) and peak to valley height (Rz) within 2 mm length of the samples were determined as the surface parameters by using a computer program. The surface roughness was measured at three different locations and was determined by obtaining an average value thereof. When comparing Comparative Example 1 with Example 1 as in Fig. 3, it can be seen that Ra has increased by the alkali treatment. This is presumably because the reticulate micro-porous structure was formed on the sample surface by the alkali treatment of Example 1. Further, when comparing Example 1 with Example 2, it can be seen that the structure of the mechanically ground surface of Example 2 has even larger surface roughness than the reticulate micro-porous structure of Example 1.

The effect of the surface treatment of the samples was evaluated by TF-XRD (RINT2000; made by Rigaku Corporation). The X-ray incidence angle to the samples was fixed to be 2˚. The composition of the outermost surface layer was analyzed by XPS (ESCA5600; manufactured by Perkin-Elmer Inc.). Monochoromatic Al $K_\alpha$ radiation (1486.6 ev) was used as the X-ray. Acquisition conditions were 13 kV, 400 W source power, and 93 eV pass energy. A photoelectron takeoff angle was set at 45˚. High-resolution scans were run for Ti, Zn, and O using an X-ray beam with a diameter of 15 nm. The XPS depth profile measurement was performed after etching with $Ar^+$ ion (an etching rate: 100 nm / min). $Ar^+$ ion etching was performed with a high-speed etching ion gun attached to UHV chamber of XPS (4 KeV). An $Ar^+$ ion irradiation angle was 90˚. An XPS spectrum was measured after $Ar^+$ ion irradiation. As a reference material, a steam sterilized Ti sample was subjected to the same XPS and TF-XRD testing.

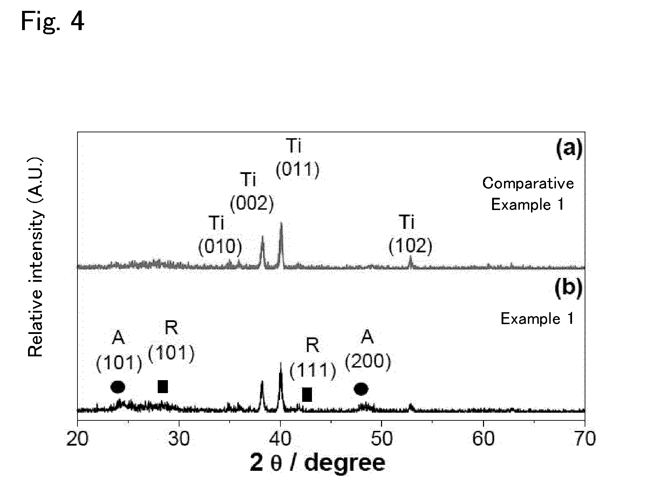

Fig. 4 shows TF-XRD patterns of the samples of Example 1 and Comparative Example 1. (a) in Fig. 4 shows the TF-XRD pattern of Comparative Example 1; (b) in Fig. 4 shows the TF-XRD pattern of Example 1. In both profiles, as the predominant peaks, $\alpha$Ti reflections of 35.1˚, 38.4˚, 40.2˚, 53.0˚, and 70.7˚ ($2\theta$, JCPD card: 44-1294) were observed. In the sample of Example 1 subjected to the alkali treatment, anatase-$TiO_2$ (101) and anatase-$TiO_2$ (200) were observed (In (b) of Fig. 4, the anatase-$TiO_2$ is indicated as "A"; rutile-$TiO_2$ is indicated as "R".). In contrast, in the sample of Comparative Example 1, amorphous oxide or oxyhydroxide of titanium were possibly present. In the sample surface of Example 1, the sodium titanate layer was not found. Further, as shown by the following EDX analysis and XPS analysis, Na content was not detected in the surface layer in any of these analyses.

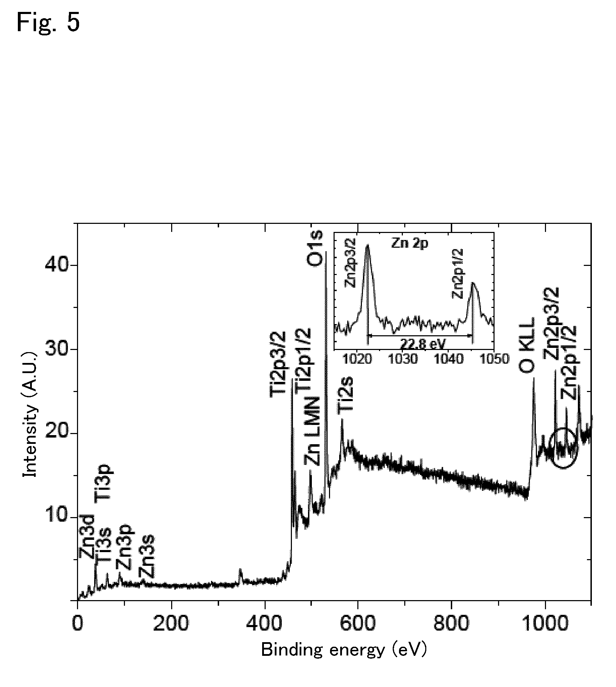

According to XPS of Comparative Example 1, C; Ca; Mg; Ti and O as a contributor to the surface oxidation were observed on the surface of the untreated Ti as impurities. It can be seen from this that the surface oxide of Comparative Example 1 is mainly $TiO_2$. On the other hand, in the sample of Example 1, Ti, Zn, and O were observed (Fig. 5). Small amounts of C, Ca, and Mg were observed; and they are seen as impurities. Further, as also stated earlier, Na was not detected on the surface of Example 1.

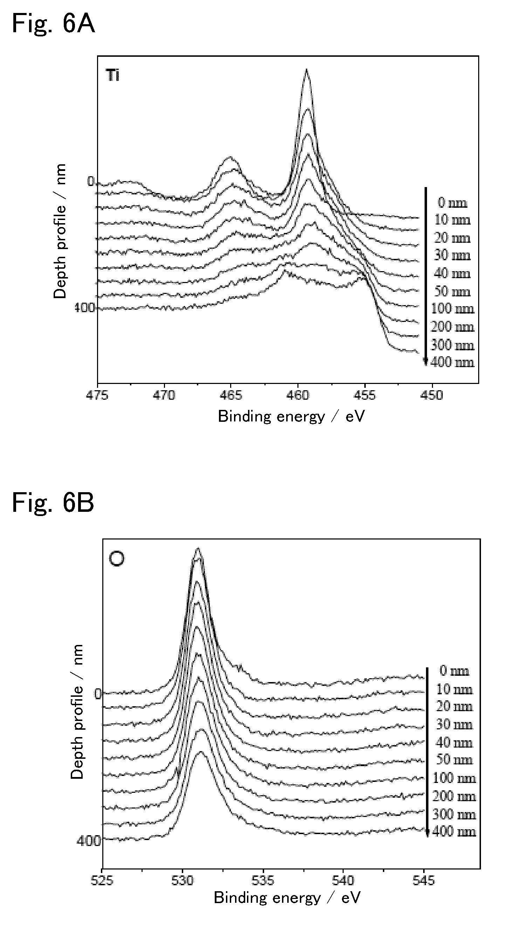

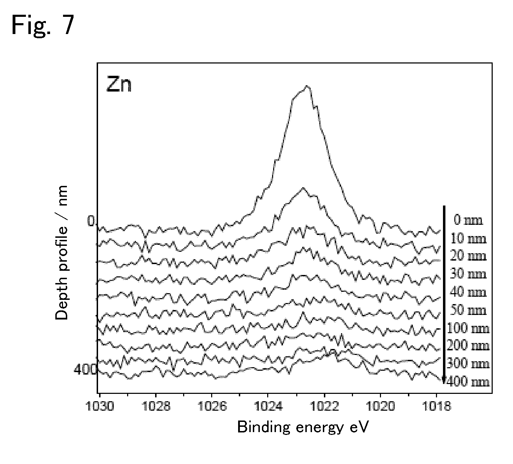

Figs. 6 and 7 show higher resolution narrow-scan spectra (50 eV pass energy) for Ti (Fig. 6A), O (Fig. 6B), and Zn (Fig.7) of the sample of Example 1. Figs. 6A, 6B, and 7 show the variation of the peaks of each of the Ti, O, and Zn, in a depth direction. Fig. 6A shows the spectra of Ti2p. The spectra consist mainly of two peaks: 459 eV (for titanium oxide Ti2p$_{3/2}$) and 464.8 eV (for titanium oxide Ti2p$_{1/2}$). These two major peaks are attributed to the tetravalent titanium such as $TiO_2$. Intensity of the tetravalent titanium ($Ti^{4+}$) decreased with an increase in the argon ion etching time. At 400 nm, a doublet corresponding to a low oxidation state of titanium was observed. Specifically, a shoulder around 455 eV for the titanium metal (Ti2p$_{3/2}$), and a shoulder around 460 ev for the titanium metal (Ti2p$_{1/2}$) were observed. It was found from these that the tetravalent titanium existed in the outermost surface layer; that there was an inclined layer where an oxygen concentration decreased toward an inner side; and that only the titanium metal existed at the depth.

Fig. 6B shows the spectra of O1s. In the spectra, the peak appeared at 531.00 eV. The O1s peak shows an asymmetrical broadening in the range of 530.4 to 535.7 eV. It can be seen from this that two types of oxygen are present in the sample surface. Further, the intensity of the O1s peak has decreased with increasing depth in the depth direction. The asymmetrical broadening of the O 1s peak is seen to be attributed to a peak at 532.4 ev related to the OH, and to a peak at around 531 eV related to the ZnO. Ususally, a binding energy in the O1s peak at 530.2 eV is related to the hexagonal $Zn^{2+}$ in the wurtzite structure. That the binding energy had a higher value in the present case indicates that the oxygen bonding is stronger than the stoichiometric Zn-O bond, thereby the Zn-O intermolecular distance being shorter than the stoichiometric Zn-O intermolecular distance. The same interpretation as this can also be found in the XPS O 1s binding energy database by NIST. It is expected from this that two types of oxygen, which are OH and ZnO are present in the outermost surface layer.

In Fig. 7, the sharp XPS peak for $Zn2p_{3/2}$ appears at 1022.4 eV, and is symmetrical, from which it can be understood that only divalent $Zn^{2+}$ is present on the surface. The Zn2p peak contributions are shown in the inset of Fig. 5. According to this, $Zn2p_{1/2}$ is at 1045.2 eV; and $Zn2p_{3/2}$ is at 1022.4 eV. It can be understood from this that divalent Zn is present in by far the outermost surface layer.

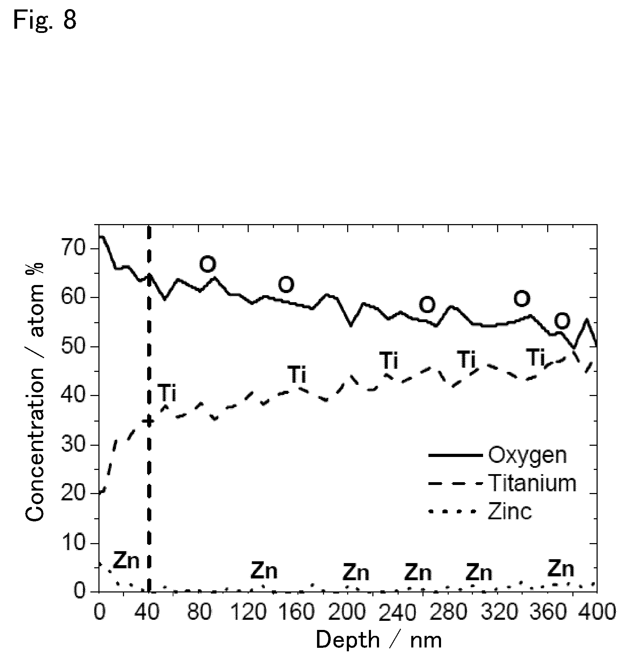

Fig. 8 shows ESCA depth profiles measured for the sample of Example 1. Looking at the oxygen profile, it can be understood that the amount of surface oxidation decreases with the increase in depth. Further, from the fact that 50 atom % of oxygen still remains at the depth of 400 nm, it can be understood that the thickness of the oxide layer is 400 nm or more. Furthermore, according to Fig.8, approximately 5 atom % of Zn is present in the outermost layer, and disappeared at the depth of approximately 40 nm. Since the carbon content disappeared after $Ar^+$ ion sputtering cleaning, in which approximately 20 nm was cut away, the carbon content is considered as a surface impurity.

The above described results of the analysis of the XPS data of Example 1, shows that "titanium oxide-Zn-O-H" is formed on the surface of the sample. The schematic diagram of the surface of the Ti base material shown in Fig. 1, was drawn on a basis of the surface structure which can be predicted from the results of the analysis of the XPS data of Example 1.

(Zn ion release test)

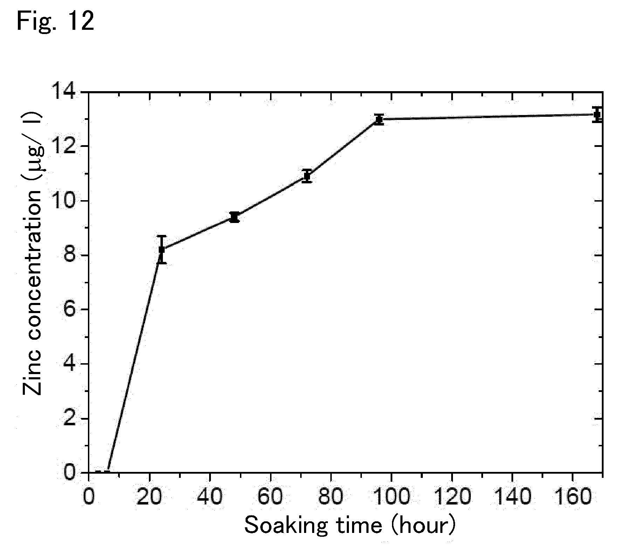

The samples of Example 1 were soaked in a physiological saline solution (0. 9% NaCl, pH 7.4) and kept in a mechanical shaker bath (37˚C) for each different time period. After that, the samples were removed, and the obtained physiological saline solution was used without dilution to measure, by ICP-AES (SPS7700; made by Seiko Instruments Inc.), a concentration of Zn ion released from each of the samples (; the emission line at 202.548 nm was used). The zinc detection limit was 0. 012 ppm. The results are shown in Fig. 12. As a result, zinc was not eluted within the first 6 hours, suggesting that this was because zinc was eluted below the ICP detection limit. With longer hours than that, the elution of zinc ion was confirmed; and a maximum of 13.2 μg/L of zinc was eluted from the samples. As stated earlier, zinc is known to promote bone formation and to inhibit bone resorption. However, this effect is attained only when a zinc concentration is extremely low. When the zinc concentration gets high, the bone formation is inhibited adversely. It was confirmed from the results shown in Fig. 12 that an amount of Zn which is tiny enough to promote bone formation was released in the samples of the present invention.

<Implant evaluation>

(Surgical placement of the implants)

The Animal Research Committee of Akita University approves the following protocols for animal experimentation. All subsequent animal experiments are strictly based on the "Guidelines for Animal Experimentation" of the University. Nine adult, male, thin, Japanese white rabbits, weighing 3.5 kg to 4.0 kg were used. These rabbits were anaesthetized with sevofrane (made by Maruishi Pharmaceutical Co.; 14 ml / kg). Each rabbit was anaesthetized with an intramuscular injection of a 3:1 mixture (4 ml) of Ketamine hydrochloride (30 mg/kg, Ketalar 200 mg; made by Sankyo Co., Ltd.) and Xylazine hydrochloride (10 mg/kg, Sedeluck; made by ZENOAQ). 1800 ml of local anesthetics (2% of lidocaine hydrochloride containing 1:80000 epinephrine (Xylocaine Poly Amp 2% (made by Fujisawa Pharmaceutical Co.))) was administered around the femur where implants were placed.

After 4, 12, and 24 weeks of the above treatment, the rabbits were anaesthetized in the same manner as above; and after the experiments, an overdose of pentobarbitalum natricum (50 mg/kg, intravenously, Nembutal (Dainippon Pharmaceutic Co., Ltd.)) was given to sacrifice the rabbits. Five cylindrical implants were used as the implant. Each implant had a length of 5 mm and a diameter of 2 mm; and was classified into four types based on whether or not an alkali treatment was carried out, and whether or not a surface grinding was performed. The alkali treatment and the surface grinding were carried out in the same manner as in the above described cases of Examples 1 and 2, and Comparative Examples 1 and 2.

(Example 3) With an alkali treatment; a smoothed surface
(Example 4) With an alkali treatment; a roughened surface
(Comparative Example 3) Without an alkali treatment; a smoothed surface
(Comparative Example 4) Without an alkali treatment; a roughened surface

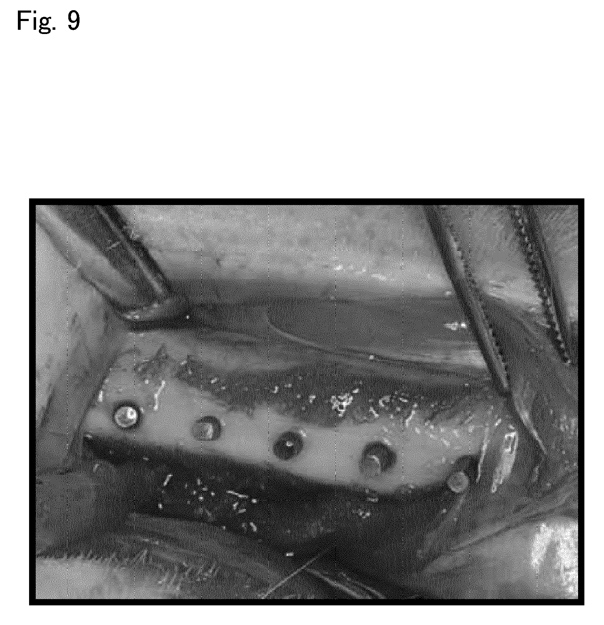

Before the surgery, the implants were sterilized by dry heat sterilization in a thermostat oven (at 180°C for 2 hours). Under sterile surgical conditions, an incision of approximately 6 cm in length was made to expose the mid-diaphyseal region of the femur.
The femoral muscles and periosteum were dissected to create a unicortical defect (a diameter of 2 mm) in a direction perpendicular to the longitudinal axis of the diaphysis. A low-speed dental drill having the same size as the implants to be inserted was used to make in the femur, a hole through the bone into the bone marrow. The hole was drilled while pouring the physiological saline solution in order to prevent overheating of the bone. After cooling and washing the holes with the physiological saline solution, the implants were inserted into the holes. Fig. 9 is a picture of the femur of the rabbit into which five implants were inserted.
Each rabbit has the five implants inserted in each of the left and right femur condyle close to the knee. After the above procedure, the muscular tissue was sutured with absorbable thread and the skin was sutured with mononylon 4-0 surgical thread. After the rabbits recovered from anaesthesia in the operation room after the operation, they were then housed individually in cages and were given food and water.

(Biomechanical testing (Measurement of the implant-bone shear strength))

Upon completion of placing the implants, after predetermined periods of time (4, 12, and 24 weeks), the resected tissue was washed and kept in ice as a soft tissue to be transported to the laboratory. The femoral tissue was cut into bone tissues (approximately 2 cm), by using a water-cooled diamond saw, each of the bone tissues containing one implant. These were kept in 0.15 M saline solution at 4°C until the next day. All the tests below were conducted with the bone specimens at a temperature equivalent to room temperature, and the bone specimens were kept moist with the saline solution.
The biomechanical tests were conducted by holding the above mentioned bone tissues (approximately 2 cm) with a metallic jig, to measure the shear strength using a computer-controlled universal testing machine (Autograph AGS-J; made by Shimadzu Corporation.) at a crosshead speed of 0.5 mm/min until the peak intensity ($F_{max}$) at a time of detachment of the implant from the bone was obtained. The above mentioned metallic jig was placed on the lower jaw of the testing machine, and a metallic load applicator having a diameter of 3 mm was fixed vertically to the upper jaw of the testing machine, ensuring that for each testing, the load imposed was parallel to the longitudinal axis of the implant.
All intensity data were converted into stress values by using the cross section of each implant. The implant-bone shear strength (MPa) is defined as:

$$\sigma = F_{max} / (\pi dt) \qquad [1]$$

In the formula [1], d is the diameter (mm) of the cylinderical implant, and t is the mean thickness (mm) of the bone tissue. The shear strength was measured at five sites for each of the samples to obtain a mean value of the five sites.
The above values of shear strength are given as the mean $\pm$ standard deviation (SD); and were assessed using one-way analysis of variance (ANOVA) at a significance level of 5%, and then compared among the samples by Tukey's test at a significance level of 5%.
The results of the biomechanical testing are shown below. All the animals (rabbits) used in the experiments tolerated the surgery and survived until the final experiment. Macroscopically, no signs of inflammation, infection, or adverse reactions were observed around any of the implants. A periosteal or endosteal callus covered the external lateral surface and intramedullary surface of all the cylindrical implants. Even after 4 weeks upon placing the implants, the implants of the present invention were firmly fixated to the bones.

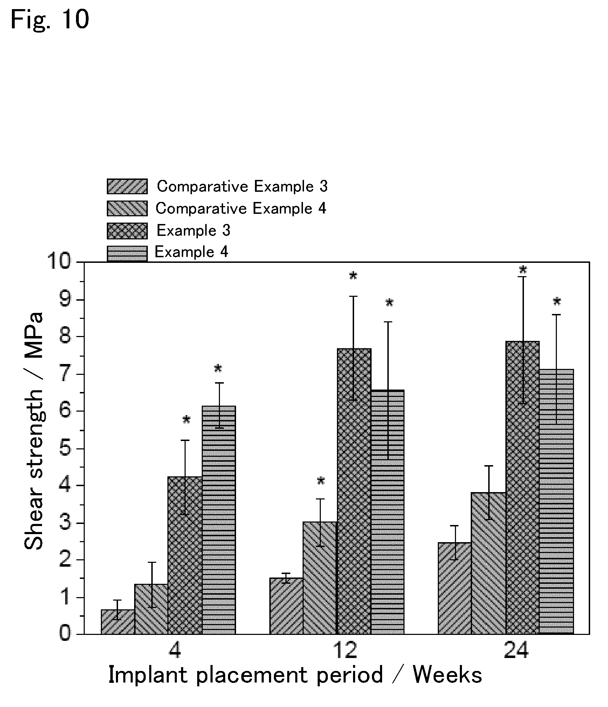

Fig. 10 shows the results of the implant-bone shear strength of Examples 3 and 4, and Comparative Examples 3 and 4 (statistical significance: $p < 0.05$). The implants of the present invention (Examples 3 and 4) better increased the implant-bone shear strength in all of the time periods (after 4, 12, and 24 weeks), compared to the implants of Comparative Examples.
Looking at the results in detail, after 4 weeks upon placing the implants, the implant-bone shear strength of Comparative Example 3 was 1 MPa or less. In contrast, the implant of the present invention likewise having a smoothed surface (Example 3) showed a shear strength of 4.23 MPa (P=0.009). In addition, the implant of the present invention having a roughened surface (Example 4) showed a shear strength of 6. 16 MPa (P=0.002). With longer time periods of implantation, the tendency of increase in the shear strength was observed. After 24 weeks upon placing the implants, the implant of Example 3 showed the maximum shear strength (9.308 MPa, P=0.001).

The Comparison between the test results of each implant-bone shear strength described in the prior art documents and the test results of the implant-bone shear strength of Example 3 of the present invention is shown in Table 1 below.

As is apparent from Table 1, in the 12 weeks when new bone formation is almost completed, when titanium was subjected to a sodium hydroxide treatment, the shear strength was approximately 3 MPa; in a case of hydroxyapatite-coated titanium practically used as a dental implant, the shear strength was approximately 3.5 MPa; and even when the surface was roughened by coating of hydroxyapatite, the shear strength was approximately 6 MPa.

In contrast, in the case of the material for a bio-implant of the present invention, the shear strength was approximately 8 MPa, which is higher than the shear strength of the implants in practical use.

[Table 1]

| Prior art documents | | Material for bio-implant | Surface treatment | Place for implantation | Time period of treatment | Implant-bone shear strength (MPa) |
|---|---|---|---|---|---|---|
| Non-Patent Document 1 | Nishiguchi S. et al, | Titanium | Alkali heating treatment and high-temperature heat treatment with sodium hydroxide | Femur of dog | 12 weeks | 3.24 |
| | Biomaterials | | | | | |
| Non-Patent Document 2 | Ozeki K. et al., | Titanium | Coating of hydroxyapatite by sputtering method | Femur of dog | 12 weeks | 3.5 |
| | Biomed Mater Eng | | | | | |
| Non-Patent Document 3 | Maxian S.H. et al., | Titanium | Plasma spray coating of crystallized hydroxyapatite on smoothed surface of titanium | Femur of rabbit | 12 weeks | 6.244 |
| | J Biomed Mater Res | | | | | |
| Non-Patent Document 4 | Hayashi K. et al., | Titanium | Coating of hydroxyapatite for obtaining roughened surface | Femur of dog | 12 weeks | 5.7 |
| | Biomaterials | | | | | |
| Example 3 | | Titanium | Alkali heating treatment or smoothed surface of titanium by using $[Zn(OH)_4]^{2-}$ complex | Femur of rabbit | 12 weeks | 7.9 |

(SEM observation)

After the tests, the surrounding bone tissue was removed completely from the retrieved implants, and was kept in a sterile plastic container filled with a saline solution. The retrieved implants and the wet tissue which is in direct contact with the implants were dehydrated with 100% acetone for 15 minutes, and dried at 180˚C. This was mounted on aluminum stub using carbon tape and was coated with a thin carbon layer to be subjected to the SEM/EDX testing.

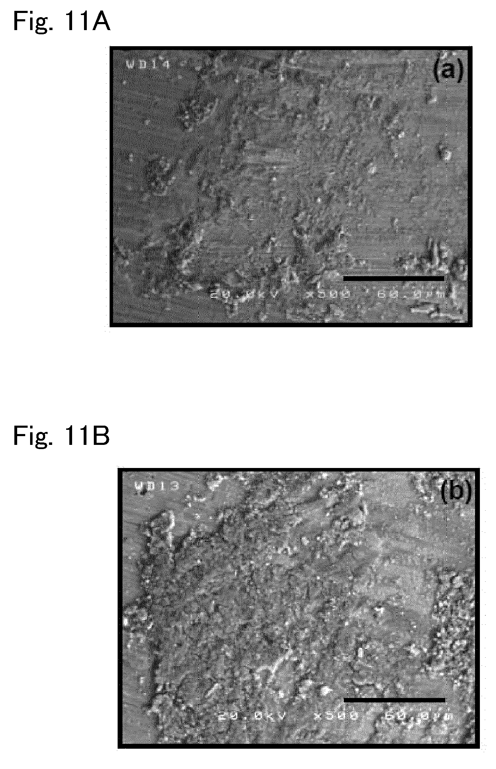

Fig. 11 is SEM images of the implant surfaces of Example 3 (Fig. 11A) and Example 4 (Fig. 11B) after the biomechanical testing. In both Examples, bone debris remained on the surfaces. On the other hand, bone debris was not seen on the implant surfaces of Comparative Examples 3 and 4. It was found from these results as well that the titanium-base material for a bio-implant of the present invention improved the implant-bone bonding.

The above has described the present invention associated with the most practical and preferred embodiments thereof. However, the invention is not limited to the embodiments disclosed in the specification. Thus, the invention can be appropriately varied as long as the variation is not contrary to the subject substance and conception of the invention which can be read out from the claims and the whole contents of the specification. It should be understood that a titanium-based material for a bio-implant and a producing method thereof with such an alternation are included in the technical scope of the invention.

Industrial Applicability

The titanium-based material for a bio-implant of the present invention can be used as an implant material in orthopedic surgery and dentistry.

**Claims**

1. A method for producing a titanium-based material for a bio-implant having a zinc functional group, which comprises a soaking step of soaking a base material made of titanium or an alloy thereof into an alkali solution containing a zinc hydroxide complex.

2. The method for producing a titanium-based material for a bio-implant having a zinc functional group according to claim 1, wherein the $OH^-$ concentration of the alkali solution is 5.0 M or more and 8.0 M or less.

3. The method for producing a titanium-based material for a bio-implant having a zinc functional group according to claim 1 or 2, wherein the soaking step is carried out by using the alkali solution having a temperature of 40˚C or more and 80˚C or less.

4. The method for producing a titanium-based material for a bio-implant having a zinc functional group according to claims 1 to 3, wherein the soaking time of the base material into the alkali solution in the soaking step is 60 minutes or more and 72 hours or less.

5. The method for producing a titanium-based material for a bio-implant having a zinc functional group according to claims 1 to 4, wherein the zinc functional group is a functional group comprising a divalent zinc atom and a hydroxyl group.

6. The method for producing a titanium-based material for a bio-implant having a zinc functional group according to claims 1 to 5, wherein the zinc hydroxide complex is $[Zn(OH)_4]^{2-}$.

7. A titanium-based material for a bio-implant comprising a base material made of titanium or an alloy thereof, wherein a surface of the base material is provided with a zinc functional group.

8. The titanium-based material for a bio-implant according to claim 7, wherein the surface of the base material made of titanium or an alloy thereof comprises a titanium oxide layer, on which the zinc functional group is provided.

9. The titanium-based material for a bio-implant according to claim 7 or 8, wherein the zinc functional group is a functional group comprising a divalent zinc atom and a hydroxyl group.

Fig. 1

-Zn-O-H
-Zn-O-H
-Zn-O-H
-Zn-O-H
-Zn-O-H
-Zn-O-H

10          20

Fig. 2A

Fig. 2B

Fig. 3

Fig. 4

Fig. 5

Fig. 6A

Fig. 6B

## Fig. 7

Fig. 8

Fig. 9

Fig. 10

Fig. 11A

Fig. 11B

Fig. 12

**INTERNATIONAL SEARCH REPORT**

| | International application No. |
|---|---|
| | PCT/JP2010/060571 |

A. CLASSIFICATION OF SUBJECT MATTER
*A61L27/00*(2006.01)i

According to International Patent Classification (IPC) or to both national classification and IPC

B. FIELDS SEARCHED

Minimum documentation searched (classification system followed by classification symbols)
A61L27/00

Documentation searched other than minimum documentation to the extent that such documents are included in the fields searched
Jitsuyo Shinan Koho          1922-1996    Jitsuyo Shinan Toroku Koho    1996-2010
Kokai Jitsuyo Shinan Koho    1971-2010    Toroku Jitsuyo Shinan Koho    1994-2010

Electronic data base consulted during the international search (name of data base and, where practicable, search terms used)
CA/MEDLINE/EMBASE/BIOSIS(STN), JSTPlus/JMEDPlus(JDreamII)

C. DOCUMENTS CONSIDERED TO BE RELEVANT

| Category* | Citation of document, with indication, where appropriate, of the relevant passages | Relevant to claim No. |
|---|---|---|
| X<br>A | JP 3-70566 A (Ellem Bioteknik AB.),<br>26 March 1991 (26.03.1991),<br>claims 6, 14, 15<br>& JP 6-4089 B          & US 5152993 A<br>& EP 409810 A3          & SE 464850 B<br>& SE 8902565 A          & CA 2021473 A | 7<br>1-6,8,9 |
| X<br>A | JP 4-54966 A (Kiyoshi INOUE),<br>21 February 1992 (21.02.1992),<br>page 2, lower left column, lines 1 to 12<br>(Family: none) | 7<br>1-6,8,9 |

☐ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
06 September, 2010 (06.09.10)

Date of mailing of the international search report
14 September, 2010 (14.09.10)

Name and mailing address of the ISA/
Japanese Patent Office

Authorized officer

Facsimile No.

Telephone No.

Form PCT/ISA/210 (second sheet) (July 2009)

**EP 2 446 906 A1**

**Patent documents cited in the description**

- JP 10179717 A
- JP 10179718 A
- JP 2000116673 A
- JP 2002102330 A

**Non-patent literature cited in the description**

- **Nishiguchi S. et al.** *Biomaterials,* 2001, vol. 22, 2525-2533
- **Ozeki K. et al.** *Bio-medical Materials and Engineering,* 2001, vol. 11, 63-68
- **Maxian SH. et al.** *Journal of Biomedical Materials Research,* 1993, vol. 27, 717-728
- **Hayashi K. et al.** *Biomaterials,* 1994, vol. 15, 1187-1191